# EUROPEAN PATENT APPLICATION

(11) **EP 4 242 299 A1**
(43) Date of publication of application: **13.09.2023**
(21) Application number: 22160552.0
(22) Date of filing: 07.03.2022
(51) Int. Cl.: C12N 7/00

(54) **SYNTHETIC VIRUS AND PROVISION THEREOF**

(71) Applicant: Technische Universität München, 80333 München (DE); von Schoenberg, Sophie, 85375 Neufahrn bei Freising (DE)
(72) Inventor: The designation of the inventor has not yet been filed
(74) Representative: Weickmann & Weickmann PartmbB

(57) **Abstract**

The present invention relates to a method for *in vitro* amplification of a linear virus genome, in particular a bacteriophage, expression and self-assembling of the virus, in particular the bacteriophage, in a cell-free expression system as well as a virus or bacteriophage provided by such methods. Further aspects of the invention relate to a synthetic bacteriophage and the use thereof.

## Description

The present invention relates to a method for *in vitro* amplification of a virus nucleic acid, in particular a bacteriophage genome, expression of the nucleic acid and self-assembling of the virus, in particular the bacteriophage, in a cell-free expression system as well as a virus or bacteriophage provided by such methods. Further aspects of the invention relate to a synthetic bacteriophage and the use thereof.

Bacteriophages (herein also called "phages") are viruses that specifically infect a host bacterium and proliferate at the expense of this host. They are composed of proteins that encapsulate a DNA or RNA genome. Bacteriophages replicate within a host bacterium by injecting their viral genetic material into the host cell effectively taking over the cells functions for the production of progeny bacteriophage leading to the rupture of the cell wall and subsequent bacterial cell death.

The biotechnological fields of application of bacteriophages are very broad and extend from evolution-based selection methods, like the evolutionary improvement of the activity of enzymes and other proteins, to phage display by which biological drug substances, e.g. therapeutic antibodies, could be generated and optimized, to the application of the bacteriophages themselves as a substitute for antibiotics.

The latter use is based on the natural characteristics of bacteriophages to specifically affect bacteria, in particular pathogenic bacteria, and kill them, i.e. to lyse them.

This approach that has been applied for a longer time to fight microbial infections gets more and more attention as the number and spread of multiresistant bacteria strains increases strongly worldwide. Methicillin-resistant *Staphylococcus aureus* (MRSA) bacteria, for example, is an increasingly common form of infection, often acquired through transmission in hospitals. MRSA infections are extremely difficult to treat using conventional antibiotics. The development of novel antibiotics is significantly slower than this development.

In the food industry bacteriophages already play an important role and are applied in this sector, for example, to detect and destroy listeria in food.

The development of phage-based therapeutics and diagnostics is, however, prevented by problems in the production and/or genetic modification of bacteriophages.

The complexity of the modification of bacteriophages is mainly due to the difficulty of changing the genome of bacteriophages.

Furthermore, the development and production of phage-based therapeutics and diagnostic is still hampered by the difficulty of a simple and safe production method for bacteriophages. In general, bacteriophages have been produced so far by *in vivo* cultivation with the appropriate bacterium. Depending on the field of application, the used bacteria may be a pathogen. This requires compliance with the appropriate safety regulations for the respective bacteria as well as the possibility to cultivate them. Also, the purification of bacteriophages produced by in vivo cultivation as such has proven to be difficult and complex.

These two main aspects of the production of bacteriophages, i.e. complexity of modification as well as simple and safe provision, have both been addressed at the same time by the methods and synthetic bacteriophages according to the present invention.

According to the present invention it is possible to carry out the production of synthetic viruses, in particular bacteriophages, completely *in vitro.* The process is based on a combination of *in vitro* methods for reproducing nucleic acids, in particular DNA, wherein the nucleic acid to be amplified can also be optionally modified, as well as completely producing bacteriophages or viruses *in vitro* by means of cell-free protein expression in a cell extract or lysate. Such inventive method may be divided into two partial aspects or methods.

Thus, a first aspect of the invention relates to a method for *in vitro* amplification of a, preferably linear, virus or bacteriophage nucleic acid, in particular a bacteriophage genome, comprising the steps of
(a) providing a virus or bacteriophage nucleic acid template to be amplified,
(b) amplification of the virus or bacteriophage nucleic acid template using polymerase chain reaction to amplify and provide different nucleic acid segments,
   wherein the primers used for at least one 5'-end of the virus or bacteriophage nucleic acid template optionally introduce nucleic acid exonuclease degradation blocking modifications, in particular phosphorothioate bonds, between the first nucleotides at the at least one 5'-end of the corresponding nucleic acid segments,
(c) recession by an 5'-3'-directional exonuclease, in particular T5 exonuclease, to provide sticky-end nucleic acid segments,
(d) annealing of the sticky-end nucleic acid segments and
(e) ligating of the annealed nucleic segments to provide amplified linear virus nucleic acids or bacteriophage nucleic acids.

A bacteriophage according to the present invention is a virus that infects and replicates within host bacteria and/or archea. Bacteriophages encompass proteins that encapsulate a DNA or RNA genome. They replicate within the host bacteria following the injection of the genome into its cytoplasm. The terms "bacteriophage" and "phage" may be applied interchangeably herein.

According to a preferred embodiment of the present invention the virus nucleic acid to be amplified, i.e. the template, may be a linear bacteriophage nucleic acid and, in particular, a bacteriophage genome. A genome to be amplified according to the present invention is preferably a linear genome. The amplified bacteriophage genome may be modified.

The nucleic acid template to be amplified may be selected from the group consisting of DNA, RNA, in particular double stranded DNA or double stranded RNA, and any variants thereof. "Variants" in particular encompasses conservatively modified nucleic acids (such as degenerate codon substitutions) and complementary sequences.

Phages are classified by the international Committee on Taxonomy of Viruses according to morphology and nucleic acid, including dsDNA-phages, ssDNA-phages, dsRNA-phages as well as ssRNA-phages. The present invention is not limited to a certain class of phages. However, dsDNA-phage are in particular preferred. Preferred dsDNA-phages comprise *Ackermannviridae*, *Autographiviridae, Chaseviridae, Demerecviridae, Drexlerviridae, Herelleviridae, Myoviridae, Podoviridae, Siphoviridae, Tectiviridae, Corticoviridae, Plasmaviridae, Lipothrixviridae, Rudiviridae, Fuselloviridae, Halspiviridae, Guttaviridae, Bicaudaviridae*, *Thaspiviridae* and *Autolykiviridae.* According to the present invention, a bacteriophage is a preferred embodiment of a virus.

According to step (a), the virus or bacteriophage nucleic acid template to be amplified is provided. Methods for providing such nucleic acid template or for providing and/or isolating the corresponding nucleic acid are well established in the art and well known to the skilled person.

Amplifying a nucleic acid template by polymerase chain reaction (PCR) according to step (b) is, in general, an established technology.

Depending on the template, respective primers have to be provided. Primer design procedures are well known to those skilled in the art. Various biotechnology companies offer tailor-made primers for all molecular biology applications and/or template-specific requirements. In principle, the primers can be designed so that the template nucleic acid, in particular the genome to be amplified, is completely amplified and/or that the individual nucleic acid segments to be provided are substantially of comparable nucleic acid lengths. Of course, the provided individual nucleic acid segments can be also of different nucleic acid length, in particular if site-specific mutations such as insertions, deletions and/or point mutations are to be incorporated.

Preferably, such nucleic acid segments have a size in the range of about 5 kbp to about 30 kbp, preferably about 5 kbp to about 25 kbp, about 5 kbp to about 15 kbp, particularly preferred about 10 kbp.

Primers used for at least one 5'-end of the template nucleic acid introduce nucleic acid exonuclease degradation blocking modifications, in particular backbone modifications. It is preferred that the primers introduce corresponding modifications at one 5'-end of the template.

According to the present invention, DNA exonuclease degradation blocking modifications, in particular backbone modifications, are modifications that lead to nuclease resistance, in particular to resistance against 5'-exonuclease. In general, such modifications can be differentiated between backbone, terminal and internal base modifications. Only few options of backbone modifications are available: phosphorothioate bonds (PTO bonds), locked nucleic acids (LNAs) and/or incorporation of aliphatic spacers are examples of backbone modifications. Terminal modifications at the 5'-end, most commonly fluorescent dyes or linker groups and/or internal base modifications at the 2'O exemplify terminal and internal base modifications, in particular in RNAs and/or mixed oligonucleotides. The use of backbone modifications is preferred.

Thus, preferred examples of backbone modifications according to the present invention include phosphorothioate bonds (PTO bonds).

It is preferable that the first 1 to 15, more preferable 1 to 10, more preferable 1 to 8 and even more preferably 1 to 5 of the first 5'-nucleotides and/or corresponding bonds of the respective primer are modified. According to other preferred embodiments of the invention 5-20, more preferably 5-18, more preferably 5-15 of the first 20 5'-nucleotides and/or corresponding bonds of the respective primer are modified or 5-15, more preferably 5-12 of the first 15 5'-nucleotides and/or corresponding bonds of the respective primer.

An insertion of DNA exonuclease degradation blocking modifications by applying the respective oligonucleotides as the primer prevents exonuclease degradation at the respective 5'-end. By introduction of such modifications undesired circulation of linear DNA fragments, in particular during litigation step (e), can be prevented.

For that purpose, the phosphodiester bonds of the first 5' nucleotides of the primer may be substituted with phosphorothioate bonds (PTO bonds). During PCR they are inserted into the DNA fragments and prevents the applied 5'-exonuclease, in particular an applied T5 exonuclease, from producing the overhangs at those positions necessary for ligation. This is a surprising result since the 5'-exonucleases are normally not stopped by the thiophosphates in the backbone of a DNA.

Thus, an especially preferred embodiment of a DNA exonuclease degradation blocking modification, i.e. circulation-blocking backbone modification is based on the introduction of phosphorothioate, i.e. the introduction of phosphorothioate bonds (PTO bonds). A DNA phosphorothioate modification is a modification on the DNA backbone, in which a non-bridging oxygen atom is replaced with a sulfur atom. The respective phosphorothioate modificated DNA may be provided by the use of phosphorothioate comprising primers during the PCR reaction. Phosphorothioate comprising primers may be provided by using phosphorothioate oligonucleotides replacing non-bridging oxygen atom on the DNA backbone with a sulfur atom when generating and/or providing respective primers.

It is preferable that the first 1 to 20, more preferably 8 to 20, more preferable 10 to 20 and even more preferably 10 to 15 phosphodiester bonds of the first 5'-nucleotides of the respective primer may be substituted with phosphorothioate bonds. According to other preferred embodiments of the invention 5-20, more preferably 5-18, more preferably 8-15 phosphodiester bonds of the first 20 5'-nucleotides of the respective primer may be substituted, or 8-15, more preferably 8-12 phosphodiester bonds of the first 15 5'-nucleotides of the respective primer.

The 5'-3'-directional exonuclease according to step (c) can be any enzyme that works by cleaving nucleotides from the 5'-end (exo) of the amplified nucleic acid template of step (b). A hydrolyzing reaction that breaks phosphodiester bonds at the 5'-end occurs. The terms 5'-3'-directional exonuclease and 5'-exonuclease may be used interchangeably herein.

By action of the 5'-exonuclease 3'-sticky-end nucleic acid segments are provided. A 3'-sticky-end nucleic acid segment shows a 3'-nucleotide overhang.

Examples of suitable 5'-exonucleases are T7-exonuclease, Exonuclease VIII (truncated), Lambda Exonuclease, T5-exonuclease and any mixtures thereof. An 5'-exonuclease which is particularly preferred is T5-exonuclease.

If during amplification step (b) backbone modifications are used, the use of T5 -exonuclease and/or Lambda exonuclease is preferred; if nucleic acid base modifications are used, the use of T7 exonuclease is preferred.

The provided sticky end nucleic acid segments are annealed in step (d). Annealing conditions and in particular the temperature depend on the template to be amplified and can in turn be determined by the person skilled in the art. The stoichiometric ratio of the sticky-end nucleic acid segments to be annealed is preferably from 1:1 to 1:5 and in particular about 1:1.

After annealing according to step (d), a polymerase, in particular a DNA polymerase such as Taq-polymerase, may fill nucleotide gaps between the annealed nucleic acid segments with free nucleotides before ligating according to step (e).

The annealed sticky-end nucleic acid segments are ligated according to step (e) to provide the amplified linear nucleic acid, in particular bacteriophage genome. In this step, the 3'-hydroxy end of one nucleic acid segment is linked to the 5'-phosphate end of another nucleic acid segment with the help of the enzyme ligase by forming a phosphodiester bond.

According to a preferred embodiment steps (a) to (e) can be carried out in a single tube reaction without isolation and/or purification of any intermediates, in particular using a one-step mastermix of enzymes.

The method described above comprising step (a) to step (e) is based on the so-called "Gibson assembly", an *in vitro* enzymatic reaction which is known in the art to ligate two or more DNA-fragments that have overlapping sequences at their ends. The basis of the Gibson assembly method is the production of DNA fragments by PCR with the primer being selected such that the DNA fragments have overlapping sequences. Then the fragments are combined by the combined activity of three enzymes. An exonuclease, preferably T5 exonuclease, generates 3'-overhangs that hybridizes with the complementary strands of the complementary sequences of the adjacent strand. A DNA polymerase fills the gaps that occurred and a ligase binds the DNA covalently. The method can also be used for site-directed mutagenesis to incorporate site-specific mutations such as insertions, deletions and/or point mutations.

However, the known Gibson assembly method bears a problem. Many phage genomes bear identical sequences on both ends of the genome that are combined by a Gibson assembly so that a circular genome is generated and a reproduction of the linear DNA in vitro is no longer possible. In order to prevent the ends of the genome, which often have overlapping sequences, from circulating the primers for the ends of the genome are modified.

This problem is, in particular, solved by the present invention by applying primers at at least one 5'-end of the virus or bacteriophage nucleic acid template, which insert exonuclease degradation blocking modifications.

If the nucleic acid template, in particular the linear genome, is to be modified, the template may be first methylated in a separate step to make it distinguishable from any new product, i.e. the amplified modified nucleic acid segment. After amplification of the template DNA via PCR in step (d) the methylated template DNA is removed by a restriction enzyme sensitive to methylation. The respective enzymes are known to a person skilled in the art and comprise, for example, Dpnl.

By this method any false-positive viruses for modification, in particular false-positive phages for modification, can be reduced to a minimum that would otherwise by generated based on the unmodified DNA. The method can be applied for any suitable modification, such as deletion, insertion and/or mutation, in particular point mutation.

Accordingly, to a preferred embodiment the inventive method comprises a step of methylating the nucleic acid template before amplification step (b), and/or a step of digesting the methylated nucleic acid template after amplification step (b).

A second aspect of the invention relates to a method for expression and self-assembling of a virus or bacteriophage in a cell-free expression system comprising the steps of
(a) providing a virus or bacteriophage nucleic acid, in particular a bacteriophage genome,
(b) providing a cell-free expression system, optionally adding of at least one protein and/or a nucleic acid encoding a protein to enhance or otherwise improve the reaction, preferably selected from the group comprising co-factors, chaperons, polymerases, transcription regulatory factors, and/or any mixtures thereof,
(c) combining the virus or bacteriophage nucleic acid and the cell-free expression system, and
(d) incubating the combined bacteriophage nucleic acid and the cell-free expression system under conditions suitable for expression and self-assembly of the bacteriophage.

According to an especially preferred embodiment, the virus or bacteriophage nucleic acid or genome is provided by the method herein described above.

A "cell-free expression system" as understood herein comprises a complete transcription and translation machinery for transcription and expression of a virus or bacteriophage nucleic acid, in particular a bacteriophage genome. The term "cell free" is understood by the person skilled in the art and refers to "substantially free of".

A preferred example for a cell free expression system is a cell lysate. Using such a cell lysate it is possible to synthesize several proteins or metabolites at the same time.

"Cell lysate" according to the present invention refers to a fluid comprising the components of cells from which it is derived after lysis. Lysis methods are known to the person skilled in the art and break down the membrane of a cell, for example, by viral, enzymatic, or osmotic mechanisms that compromise its integrity. Such cell lysate is essentially void of intact cells, i.e. cell-free. The terms "cell lysate" and "cell extract" can be used herein interchangeably.

After purification this lysate is free of host DNA and makes an expression of the desired protein possible by the external addition of DNA, in particular a amplified bacteriophage genome as herein described. Thus, the used cell lysate is preferably free of nucleic acids, in particular DNA, and/or membranes from the cells of which it is derived.

Cell lysates used according to the present invention may be derived from microorganisms, in particular bacteria such as pathogenic bacteria and E. *coli,* yeast, insects, mammals and/or plants, in particular such as wheat or rice, or may be even artificial. "Microorganism" refers to a bacterium or an archaeon. Preferably, the microorganism is a bacterium.

A number of cell-free expression systems are known to a person skilled in the art und may be applied according to the present invention. For example, the "PURE" system consists of several isolated proteins (Shimizu et al.) while untreated cell lysates (crude extracts) such as of *E. coli* include almost all intracellular proteins, even those that are not necessary for expression.

The use of *E. coli* lysate, in particular crude *E. coli* lysate, is a further preferred embodiment. A preferred example of crude *E. coli* lysate is *E.coli* S30 cell extract produced by a method based on the protocol described in E. Falgenhauer et al. (Falgenhauer, S. von Schönberg, C. Meng, A. Muckl, K. Vogele, Q. Emslander, C. Ludwig, F. C. Simmel, ChemBioChem 2021, 22, 2805).

External factors such as energy carriers, co-factors, amino acids, polymerases, transcription regulatory factors , chaperons may be added to enhance or otherwise improve the reaction. Such factors may be added as proteins and/or as nucleic acid encoding a respective protein.

If the cell-free lysate to be used is derived from a cell or microorganism which is different to the natural host of the virus or bacteriophage, at least one bacteriophage-host specific expression factor and/or a nucleic acid encoding the at least one bacteriophage-host specific expression factor, preferably a transcription factor, can be added, if necessary.

According to one embodiment of the invention, at least one nucleotide sequence encoding the at least one bacteriophage-host specific expression factor or any other protein preferably selected from the group comprising co-factors, chaperons, polymerases, transcription regulatory factors is cloned into the cell strain used to provide the cell lysate. The nucleotide sequence is expressed within the cell before lysis. Thus, after lysis the cell lysate already contains all nucleic acids and/or proteins necessary for the amplification of the virus or bacteriophage, in particular at least one host specific expression factor or any other protein selected from the group comprising co-factors, chaperons, polymerases, transcription regulatory factors and/or any mixture thereof.

The method can also be used to produce a modified bacteriophage where at least one protein of interest is modified or added. The modified or additional protein of the bacteriophage can be provided by adding that protein to the cell-free expression system and/or by adding a nucleic acid encoding that protein. This protein itself can be of natural amino acid sequence or artificially modified for optimization.

According to a preferred embodiment a modified bacteriophage of the invention may be produced by applying at least one step selected of the group:
(i) providing a modified bacteriophage genome,
(ii) adding a modified or unmodified protein to be assembled into the bacteriophage that differs from the corresponding protein in that bacteriophage,
(iv) adding a nucleic acid encoding for a modified or unmodified protein to be assembled into the bacteriophage where the protein differs from the corresponding protein in that bacteriophage,
(v) a nucleic acid molecule that suppresses the expression of a protein of a bacteriophage, and/or
(vi) any combination of steps (i)-(v) thereof.

The cell-free synthesis of proteins, i.e. outside living cells, has several advantages over cellular expression. This is all the more true if proteins are expressed that are toxic for the bacteria or non-natural amino acids shall be inserted into the proteins.

The production of bacteriophages in a cell-free lysate may also by carried out without any bacteriophage host bacteria, which makes it possible to produce them locally, e.g. in a laboratory without biosafety-level. At the same time the amount of toxic by-products that are produced by a method according to the present invention is reduced, since lysed pathogenic host bacteria are not present.

Another advantage is that if the *in vitro* method is based on a cell-free lysate only the genome of the added virus or bacteriophage is applied and thus, a pure solution of viruses or bacteriophage can be produced, without any pro-viruses or pro-phages. The cell-free expression in combination with the *in vitro* reproduction of nucleic acids and in particular the genome represents a broadly applicable method that accelerates the application of bacteriophages in research and development and biomedical applications.

According to a preferred embodiment expression and and/self-assembly during incubating step (d) may be monitored.

Thus, a further aspect of the invention relates to an all *in vitro* method for the amplification and provision synthetic viruses, in particular bacteriophages, i.e. fully *in vitro* generated viruses and/or bacteriophages. Such all *in vitro* method relates to a combination of the method for *in vitro* amplification, expression and self-assembling, as described herein.

Accordingly the all *in vitro* method comprises
(I) *in vitro* amplification of a linear virus or bacteriophage nucleic acid, in particular bacteriophage genome, comprising the steps of
   (a) providing a virus or bacteriophage nucleic acid template to be amplified,
   (b) amplification of the virus or bacteriophage nucleic acid template using polymerase chain reaction to amplify and provide different nucleic acid segments,
      wherein the primers used for at least one 5'-end of the virus or bacteriophage nucleic acid template introduce nucleic acid circulation blocking modifications, in particular phosphorothioate bonds, between the first nucleotides at the at least one 5'-end of the corresponding nucleic acid segments,
   (c) recession by an exonuclease, in particular T5 exonuclease, to provide sticky end nucleic acid segments,
   (d) annealing of the sticky nucleic acid segments and
   (e) ligating of the annealed nucleic segments to provide amplified linear virus nucleic acids or bacteriophage nucleic acids and
(II) expression of the provided amplified linear virus nucleic acids or bacteriophage nucleic acids and self-assembling of the expressed virus or bacteriophage proteins in a cell free expression system comprising the steps of
   (a') providing a virus or bacteriophage nucleic acid, in particular a bacterio-phage genome,
   (b') providing a cell free expression system, optionally adding of at least one protein and/or a nucleic acid encoding a protein to enhance or otherwise improve the reaction, preferably selected from the group comprising co-factors, chaperons, polymerases, transcription regulatory factors, and/or any mixtures thereof,
   (c') combining the virus or bacteriophage nucleic acid and the cell free expression system, and
   (d') incubating the combined bacteriophage nucleic acid and the cell free expression system under conditions suitable for expression and self-assembly of the bacteriophage.

Using such method wildtype viruses and/or modified phages can be provided. According to the present invention, a "modified phage" can be modified on the genome level and/or on proteome level.

By the present invention it could be shown for the first time that it is possible to produce synthetic phages *in vitro* completely, which accelerates and facilitates the production as well as the modification of viruses and/or bacteriophages.

The concentration of bacteriophages generated *in vitro* is so high that the corresponding composition can directly be applied in a therapy with phages without a further concentration step. However, if needed, a further concentration step, in particular after purification, is of course also within the scope of the present invention.

A further aspect of the invention relates to a synthetic virus or bacteriophage provided by the *in vitro* methods herein. The synthetic bacteriophage may have the same lytic activity and/or the same phenotypic characteristics as the corresponding wild-type bacteriophage has. According to a preferred embodiment such phage has a modified genome and/or proteome.

A further aspect of the invention relates to the use of bacteriophages provided as described herein for use in medicine, chemistry, biotechnology, agriculture and/or food industry. Such uses are not limited to bacteria related fields and comprise, for example, the use as vaccine, for example against infections as well as tumors, and the use as a delivery vehicle for any kind of drug, in particular anti-cancer drugs, to a target cell, in particular a human target cell.

The use in bacteria related fields is, however, preferred. Bacteriophages may in particular be used as bacterial population control alternatives to antibiotics. They are much more specific than antibiotics and are typically harmless not only to the host organism but also to other beneficial bacteria, such as the gut microbiota, as they are very selective in the strains of bacteria they are effective against. Thereby the chances of opportunistic infections are significantly reduced. Advantages include further reduced side-effects and reduced risk of the bacterium's developing resistance. Because phages replicate *in vivo*, a small effective doses can be used.

These unique properties make them highly promising antimicrobials.

Accordingly the synthetic bacteriophages provided herein may be part of a pharmaceutical composition. Such a composition may comprise (i) at least one bacteriophage strain capable of producing a lytic infection and (ii) a pharmaceutically acceptable carrier. Of course, such composition may comprise different bacteriophage strains, i.e. phage mixtures.

The pharmaceutical compositions may be used in combination with antibiotics for the purpose of treating bacterial infections and/or to treat antibiotic resistant bacteria. In particular, bacteriophages tend to be more successful than antibiotics where there is a biofilm covered by a polysaccharide layer, which antibiotics typically cannot penetrate. According to a preferred embodiment, the composition may be freeze dried.

"Pharmaceutically acceptable carrier" relates to pharmaceutically-acceptable, fillers or diluents used to formulate pharmaceutical compositions for animal or human administration. The pharmaceutical compositions may further comprise pharmaceutically acceptable auxiliary agents, and optionally other therapeutic agents. In particular for oral administration it is preferred to add an antacid, i.e. a substance which neutralizes stomach acidity, thereby increasing the number of phages surviving passage through the stomach.

The dose and regimen of administration of a pharmaceutical composition will necessarily be dependent upon the therapeutic effect to be achieved (e.g. treatment of IBD) and may vary with the particular bacteriophage strains in the composition, the route of administration, and the age and condition of the individual subject to whom the pharmaceutical composition is to be administered.

If the pharmaceutical composition is to be administered with one or more antibiotics, it may be simultaneously, separately or sequentially administered.

Pharmaceutical compositions and routes of administration include those suitable for or via oral (including buccal, sublingual and intraorbital), rectal, nasal, topical (including transdermal), ocular, vaginal, bronchial, pulmonary or parenteral (including subcutaneous, intramuscular, intravenous, intradermal, intraperitoneal, intrapleural, intravesicular and intrathecal) administration or administration via an implant.

In particular a freeze-dried composition may be administered orally, preferably on form of a pill.

Agriculture comprises plant agriculture as well as animal agriculture, wherein bacteriophages can replace or complement antibiotics and/or pesticides.

Bacterial pathogens are associated with a couple of plant diseases which can also be treated with synthetic bacteriophages provided herein. Non limiting examples for bacterial plant diseases which might be treated are leaf blight on onion, potato scab or soft rot of potato, bacterial wilt on tobacco, citrus bacterial spot or citrus canker of citrus, fire blight on apple, black rot of cabbage, soft rot of calla lilies, bacterial wilt of tomato and bacterial spot of peach.

The use of the inventive synthetic bacteriophages in animal agriculture is exemplified by the use against *Salmonella*, pathogenic *E. coli*, *Clostridium*, and *Campylobacter* for the poultry industry and against *Salmonella* and pathogenic E. *coli* for the pig industry.

Phages provided herein may also be used to safen food products, and to forestall spoilage bacteria. Since 2006, the United States Food and Drug Administration (FDA) and United States Department of Agriculture (USDA) have approved several bacteriophage products. For example, LMP-102 (Intralytix) was approved for treating ready-to-eat (RTE) poultry and meat products. In that same year, the FDA approved LISTEX using bacteriophages on cheese to kill Listeria monocytogenes bacteria.

With regard to biotechnology related uses, the methods described as well as the bacteriophages provided herein can, for example, be used for phage display related application.

The invention further includes a kit comprising a pharmaceutical composition of the invention and instructions for the use of the composition for a use as hereinbefore described, optionally together with packaging material.

Further aspects relate to the use of bacteriophages provided herein in methods for the prevention and or treatment of a bacterial infection as well as for avoiding bacterial growth.

### Figures

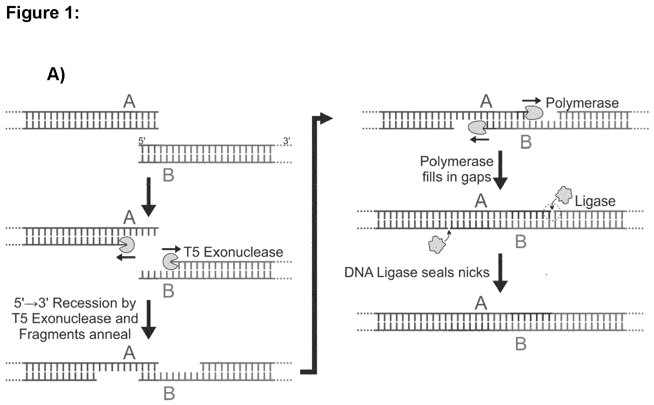

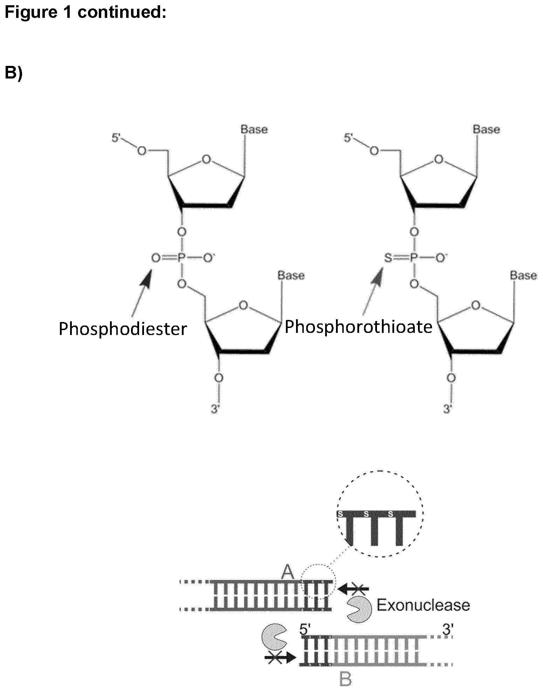

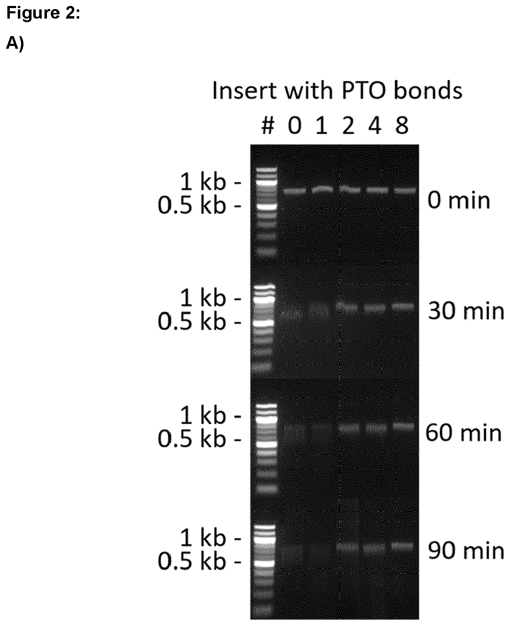

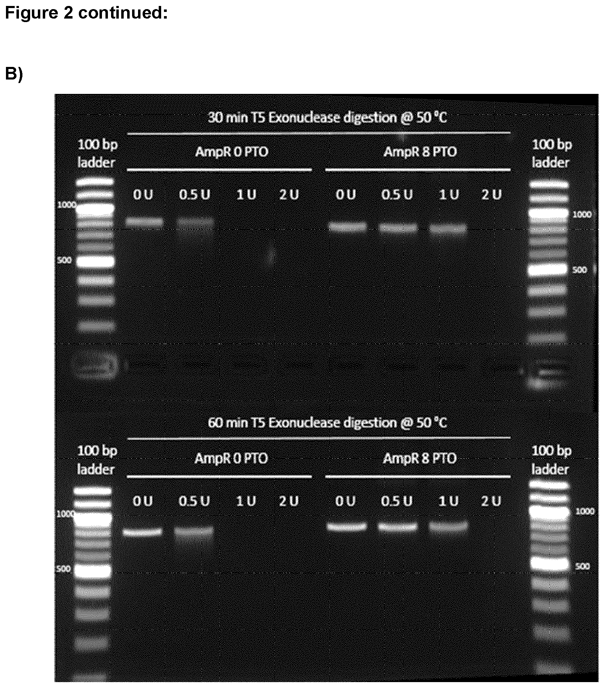

- **Figure 1**:: In vitro DNA manipulation
(A) Gibson assembly
(B) PTO (Phosphorothioate) bonds to protect from T5 exonuclease
- **Figure 2:**: Inhibition of T5 exonuclease by PTO bonds. The enzymatic assay shows digestion by T5 exonuclease, whereby 8 PTO bonds offer protection from T5 exonuclease recession.

### Example

### Producing of E.coli phages according to the invention

The T7 *E.coli* phage has a genome that is 40 kbp in size with overlapping sequences at its ends. This genome was completely produced synthetically with the method according to the present invention.

As a first step the template was methylated with DAM-methylase.

After that four nucleic acid segments about 10 kbp in size were amplified in a PCR reaction The first and the last primer at the genome were modified in that the first eight base pairs showed phosphorothioate bonds in the backbone and, unlike the other primers, only a phosphodiester backbone. In detail, the primers used at both ends of the genome had phosphorothioate bonds (in the backbone) between the first 8 bases, the remaining nucleotides were linked via (regular) phosphodiester bonds.

After the PCR reaction the methylated template DNA was removed by the enzyme Dpnl and primers in excess and the removed template were removed by a PCR-clean-up kit.

The four DNA fragments were added in a stoichiometric ratio of 1:1:1:1 and assembled with a Gibson mastermix.

Afterwards the DNA was added to a cell-free expression system. The cell-free expression system was *E.coli* S30 cell extract that was produced according to the protocol of E. Falgehnauer et al. (E. Falgenhauer, S. von Schönberg, C. Meng, A. Muckl, K. Vogele, Q. Emslander, C. Ludwig, F. C. Simmel, ChemBioChem 2021, 22, 2805).

This mixture was incubated for 8 h at 29°C.

Then the bacteriophages that were completely produced synthetically were verified via a plaque assay.

## Claims

1. Method for *in vitro* amplification of a virus or bacteriophage nucleic acid, in particular a bacteriophage genome, comprising the steps of
(a) providing a virus or bacteriophage nucleic acid template to be amplified,
(b) amplification of the virus or bacteriophage nucleic acid template using polymerase chain reaction to amplify and provide different nucleic acid segments,
wherein the primers used for at least one 5'-end of the virus or bacteriophage nucleic acid template optionally introduce nucleic acid exonuclease degradation blocking modifications, in particular phosphorothioate bonds, between the first nucleotides at the at least one 5'-end of the corresponding nucleic acid segments,
(c) recession by an 5'-3'-directional exonuclease, in particular T5 exonuclease, to provide sticky end nucleic acid segments,
(d) annealing of the sticky nucleic acid segments and
(e) ligating of the annealed nucleic segments to provide amplified linear bacteriophage nucleic acids.

2. The method of claim 1,
wherein the bacteriophage nucleic acid is selected from the group consisting of DNA, RNA and variants thereof.

3. The method of claim 1 or 2,
wherein the different nucleic acid segments are of comparable nucleic acid length, preferably about 5 kbp to about 30 kbp, in particular about 10 kbp.

4. The method according to any of claims 1 to 3,
wherein stoichiometric ratio of sticky nucleic acid segments to be annealed in step (d) is 1:5, in particular 1:1.

5. The method of any of claims 1 to 4,
wherein the provided amplified virus or bacteriophage nucleic acid is modified, in particular modified by deletion, insertion and or point mutation of nucleic acids.

6. The method of claim 5,
wherein the method comprises
a step of methylating the virus or bacteriophage nucleic acid template before amplification step (b), and
a step of digesting the methylated virus or bacteriophage nucleic acid template after amplification step (b).

7. Method for expression and self-assembling of a virus or bacteriophage in a cell free expression system comprising the steps of
(a) providing a virus or bacteriophage nucleic acid, in particular a bacteriophage genome, preferably a virus or bacteriophage nucleic acid or genome provided by the method according to any of claims 1 to 6,
(b) providing a cell-free expression system, optionally adding of at least one protein and/or a nucleic acid encoding a protein to enhance or otherwise improve the reaction, preferably selected from the group comprising co-factors, chaperons, polymerases, transcription regulatory factors, and/or any mixtures thereof,
(c) combining the virus or bacteriophage nucleic acid and the cell-free expression system, and
(d) incubating the combined virus or bacteriophage nucleic acid and the cell-free expression system under conditions suitable for expression and self-assembly of the bacteriophage.

8. The method of claim 7,
wherein the cell-free expression system is host independent and preferably selected from cell lysates or artificial expression systems.

9. The method according to claim 8,
wherein the cell lysates are derived from microorganisms, in particular E. *coli,* yeast, insects, mammals, plants and/or are artificial.

10. The method according to any of claims 7-9, wherein a modified bacteriophage is produced by applying at least one step selected of the group:
(i) providing a modified bacteriophage genome,
(ii) adding a modified or unmodified protein to be assembled into the bacteriophage that differs from the corresponding protein in that bacteriophage,
(iv) adding a nucleic acid encoding for a modified or unmodified protein to be assembled into the bacteriophage where the protein differs from the corresponding protein in that bacteriophage,
(v) a nucleic acid molecule that suppresses the expression of a protein of a bacteriophage, and/or
(vi) any combination of steps (i)-(v) thereof.

11. Virus, in particular bacteriophage, provided by any of claims 1 to 10.

12. An all *in vitro* method for the amplification and provision of synthetic bacteriophages.

13. Virus, in particular bacteriophage, according to claim 11 or 12 for use in medicine, chemistry, biotechnology, agriculture and/or food industry.

14. Virus, in particular bacteriophage, of according to for use in a method for the prevention and or treatment of a bacterial infection.

15. Use of a virus, in particular a bacteriophage according to claim 11 or 12 for avoiding bacterial growth.
